# EUROPÄISCHE PATENTANMELDUNG

(11) **EP 0 825 440 A1**
(43) Veröffentlichungstag der Anmeldung: **25.02.1998**
(21) Anmeldenummer: 97111422.8
(22) Anmeldetag: 07.07.1997
(51) Int. Cl.: G01N 33/44, G01N 30/00

(54) **Verfahren zur Untersuchung von Mischungen aus Bitumen und Polymeren**

(30) Priorität: 22.08.1996 DE 19633804
(71) Anmelder: HÜLS AKTIENGESELLSCHAFT, 45764 Marl (DE)
(72) Erfinder: Herrmann, Christoph, Dr., 45768 Marl (DE); Bickert, Peter, Dr., 48249 Dülmen (DE)

(57) **Zusammenfassung**

Qualitative und quantitative Analysemethoden für Bitumen/Polymer-Mischungen sind bisher nur für spezielle Polymere ausgearbeitet worden. Das erfindungsgemäße Pyrolyse-Gaschromatographie-Verfahren bietet erstmalig eine einfache, breite Anwendbarkeit.

Qualitative und quantitative Bestimmung des Polymergehalts in Bitumen/Polymer-Mischungen.

## Beschreibung

Die Erfindung betrifft ein Verfahren zur qualitativen und/oder quantitativen Bestimmung des Polymergehaltes in Mischungen aus Bitumen und Polymeren.

Bitumen fällt als Rückstand bei der destillativen Aufarbeitung von Erdölen an. Der chemische Aufbau von Bitumen ist sehr komplex. Es handelt sich um ein Gemisch verschiedenster Kohlenstoffverbindungen, dessen Zusammensetzung aus paraffinischen, naphthenischen und aromatischen Anteilen je nach Ausgangsöl und Raffination stark schwankt.

Es ist bekannt, Bitumen z.B. in Dach- und Dichtungsbahnen sowie in Asphaltestrichen einzusetzen. Zur Verbesserung der Eigenschaften in diesen Anwendungsgebieten wird das Bitumen mit unterschiedlichen Polymeren modifiziert.

Die wichtigsten Polymergruppen zur Bitumenmodifizierung sind:
- Polyethen und Copolymere (PE, LDPE)
- ataktisches Polypropen (APP)
- ataktische Terpolymere aus Ethen, Propen und Buten (APAO)
- Styrol-Butadien-Copolymere/-Blockcopolymere (SBR, SBS)
- Ethyl-Butylacrylat-Copolymer (EBC)
- Ethen-Vinylacetat-Copolymer (EVA).

Die anwendungstechnische Prüfung dieser Bitumen/Polymer-Verschnitte im Zuge von z. B. Qualitätseingangskontrollen kann mittels Viskosität, Penetration und Zugfestigkeit erfolgen. Bei bekanntem Zusammenhang zwischen Zusammensetzung und Verschnitteigenschaften kann so auf die eingesetzte Polymerkomponente zurückgeschlossen werden. Dies ist jedoch trotz des Prüfumfanges nicht eindeutig; daher ist ein direkter Nachweis des Modifiers vorteilhafter.

Für die qualitative und quantitative Analytik der Polymer-Bitumen-Mischungen ist bisher nur eine begrenzte Anzahl von Methoden beschrieben worden (A. Samadijavan et al., Bitumen 1/96, Seiten 13 - 20).

Extraktive Methoden sowie die Lösungs-Fällungs-Fraktionierung setzen unterschiedliche Lösungseigenschaften der Mischungskomponenten voraus und wurden nur für spezielle Polymere ausgearbeitet, z. B. für SBR (A. Samadijavan et al., Bitumen 1/96, Seiten 13 - 20). Die naß-chemischen Verfahren sind zudem sehr aufwendig.

Als eine instrumentelle Methode der Polymerbestimmung wird die Infrarot-Spektroskopie eingesetzt, gegebenenfalls nach einem anreichernden Extraktionsschritt. Dies ist jedoch nur möglich, wenn die Polymerabsorptionsbanden nicht mit den Bitumeneigenabsorptionen überlagern. Es sind spezielle Verfahren für LDPE, EVA und SBS bekannt (L.H. Lewandowsky, Rub.Chem.Technol. 67 (1994), Seiten 447 - 478).

Eine weitere Methode ist die Gelpermeationschromatographie (GPC), bei der die Mischungen in Lösung aufeine Säule gebracht werden, die die Komponenten aufgrund ihrer unterschiedlichen Molmassen auftrennt. Diese Methode wurde speziell für amorphe Polyolefine ausgearbeitet, die aufgrund ihrer Ähnlichkeit mit Bitumen nur unzureichend mit den anderen genannten Methoden bestimmt werden können. Zur vollständigen Lösung müssen jedoch hohe Temperaturen angewandt werden, bei denen auch die GPC durchzuführen ist (K. ObA et al., Materials and Structures 183 (1995), Seiten 534 - 544).

Mischungen von Bitumen mit Polymeren mit kristallinen Anteilen können auch mit der Dynamischen Differenzkalorimetrie untersucht werden. Da Bitumen mit steigender Temperatur eine relativ konstante Änderung seiner Eigenschaften zeigt, werden Polymere mit Umwandlungstemperaturen detektiert (K.Oba, Dissertation, Bull. 168 der Division of Building Technology, Stockholm 1994).

Zur Qualitätsbeurteilung der modifizierten Bitumen besteht daher ein Bedarf für ein möglichst universelles Untersuchungsverfahren, mit dem die polymere Verschnittkomponente auf einfache Weise spezifisch nachgewiesen und ihre Konzentration vorzugsweise zugleich quantitativ bestimmt werden kann.

Überraschenderweise wurde gefunden, daß diese Aufgabe gemäß den Patentansprüchen mittels Pyrolyse-Gaschromatographie gelöst wird, indem eine Probe der Bitumen/Polymer-Mischung in inerter Atmosphäre pyrolysiert wird und die Pyrolyseprodukte on-line gaschromatographisch analysiert werden.

Die Hauptkomponente der zu untersuchenden Proben besteht aus Bitumen. Im allgemeinen beträgt der Bitumenanteil ca. 60 bis 98 Gew.-%. Ausgangsprodukt für die Gewinnung von Bitumen sind - wie erwähnt - Erdöle unterschiedlichster Vorkommen, bei deren Destillation es als Rückstand zurückbleibt. Bitumen ist daher ein Gemisch verschiedener Kohlenwasserstoffe; die Zusammensetzung aus paraffinischen, naphthenischen und aromatischen Anteilen schwankt je nach Ausgangsöl und Herstellungsverfahren. Der chemische Aufbau ist sehr komplex. Es können aufgrund unterschiedlicher Lösungseigenschaften zwei Fraktionen charakterisiert werden: höhermolekulare Asphaltene und niedermolekulare, ölige Maltene; die Asphaltene haben gegenüber den Maltenen einen stärker aromatischen Charakter (K.-H. Güsfeldt, Ullmann Band 8, 4. Aufl., 1974, Seiten 527 - 541).

Die Bitumen werden charakterisiert u. a. durch Penetration (DIN 52 010) und Erweichungspunkt (DIN 52 011). Zur Bezeichnung des Typs wird dem Namen der Penetrationswert nachgestellt, z. B. Bitumen B 80.

In der Pyrolyse wird diese komplexe Struktur des Bitumens zerbrochen zu überraschenderweise einfachen Bruchstücken. Für Bitumen unterschiedlicher Zusammensetzung tritt unerwarteterweise stets ein einfaches Fragmentierungsmuster aus homologen Kohlenwasserstoffreihen CₙHₓ mit der natürlichen Zahl n ≥ 1 und der natürlichen Zahl x mit einem Wert von 2n - 2 bis 2n + 2 auf, das vergleichbar mit z B. dem von Paraffinen und Polyethenen ist (E. Kiran et al., J. Appl. Poly. Sci. 20 (1976), Seiten 2045 - 2068). Die polymeren Modifier fragmentieren hingegen entsprechend ihrem Aufbau aus verschiedenen Monomeren in Bruchstücke zu ebenfalls homologen Reihen, deren Retentionszeiten sich im Chromatogramm von denen der Bitumenbruchstücke unterscheiden. Geeigneterweise nutzt man daher erfindungsgemäß zur Identifizierung des Polymers ein bei der Pyrolyse bevorzugt gebildetes, von den Bitumenbruchstücken deutlich getrenntes Hauptbruchstück des Polymers und setzt seinen Chromatogrammausschlag vorzugsweise ergänzend zur Quantifizierung ins Verhältnis zu dem Ausschlag eines eindeutigen Bitumenbruchstücks. Anhand dieses Verhältnisses kann die Zusammensetzung durch Vergleich mit Kalibriergemischen von Bitumen mit dem entsprechenden Polymer auch quantitativ ermittelt werden.

Polymere liefern in der Pyrolyse stets ihre Monomeren und deren Oligomere. Hieraus folgt lediglich eine Einschränkung in der universellen Anwendbarkeit des erfindungsgemäßen Verfahrens: Homopolyethylene können aufgrund des gleichen Fragmentierungsmusters wie bei Bitumen nicht bestimmt werden, wohl aber Copolymere mit den höheren Olefinen (C_{≥3}) als Comonomereinheiten. Darüber hinaus ist das erfindungsgemäße Verfahren jedoch auf alle Polymergruppen zur Bitumenmodifizierung anwendbar. Es können auch Bitumen/Polymer-Mischungen mit mehreren Polymeren als Modifier untersucht werden, also z. B. Bitumen/Polymer-Mischungen mit Modifier-Mischungen.

Das erfindungsgemäße Verfahren wird üblicherweise wie folgt durchgeführt:

Die Proben werden zunächst in einem Pyrolyseofen fragmentiert. Es können unterschiedliche Arten des Energieeintrages angewandt werden, z. B. Curie-Punkt-Drähte, Heizdrähte und Platin-Schiffchen in einem Heizdraht-, Laser- oder Mikrowellenofen. Bevorzugt wird ein Curie-Punkt-Pyrolysator eingesetzt. Die Pyrolysedauer beträgt im allgemeinen < 10 Sekunden, vorzugsweise 5 bis 0,01 Sekunden. Die Pyrolyse wird im allgemeinen in einem Temperaturbereich von 400 - 900 °C durchgeführt, vorzugsweise in einem Bereich von 500 - 800 °C. Im allgemeinen wird ein Trägergasstrom über die zu pyrolysierende Probe geleitet; geeigneterweise handelt es sich direkt um den Trägergasstrom des angeschlossenen Gaschromatographen, z B. um Stickstoff oder Wasserstoff oder Edelgas wie Helium , so daß die Pyrolysebruchstücke direkt in den Gaschromatographen getragen werden.

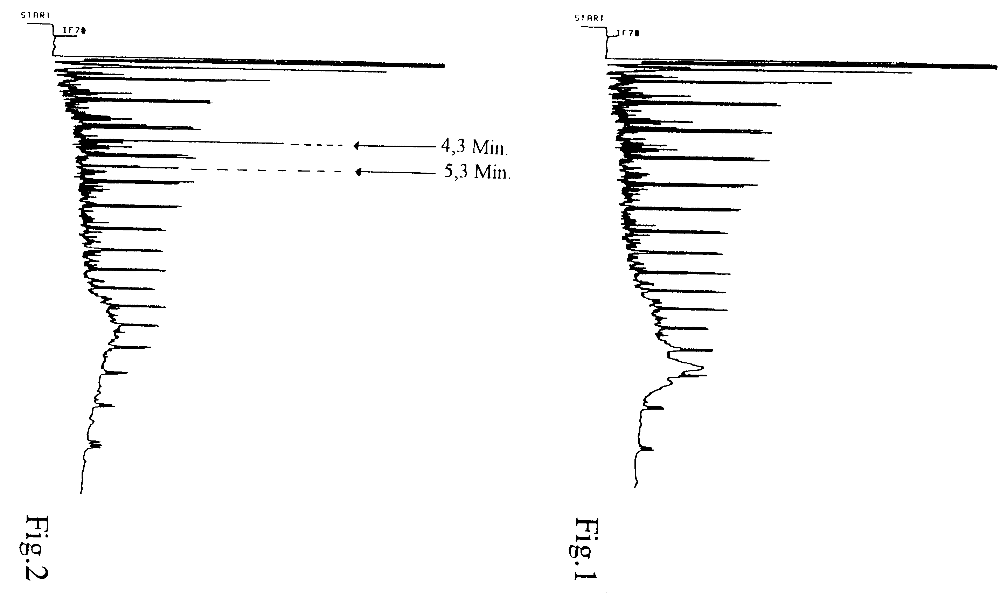

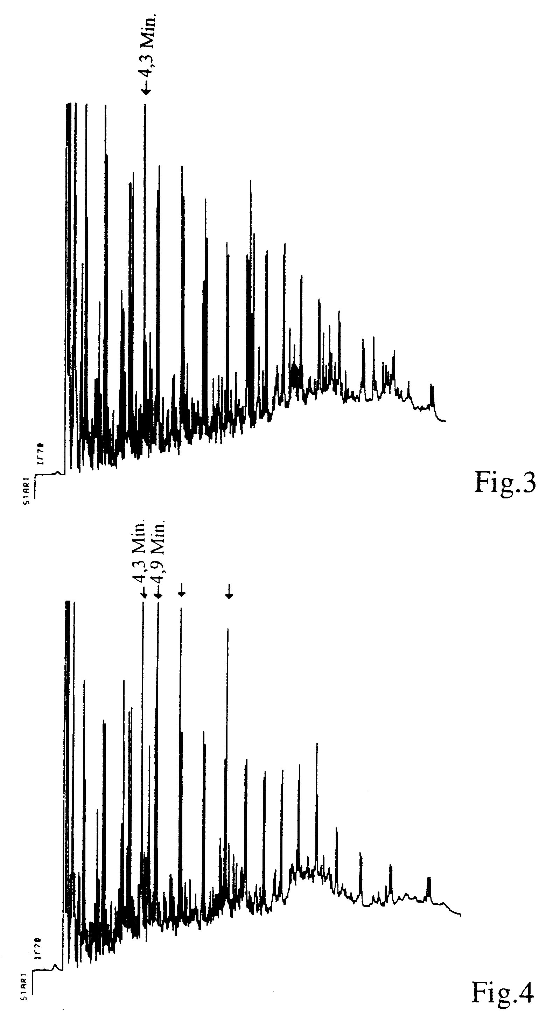

In der on-line Gaschromatographie werden im allgemeinen Kapillarsäulen eingesetzt, vorzugsweise eine Glaskapillare mit der stationären Phase 5 % Diphenyldimethylpolysiloxan, einer Länge von 25 m, einem Innendurchmesser von 0,32 mm und einer Filmdicke von 0,52 µm. Die Detektion erfolgt vorzugsweise mit einem Flammenionisationsdetektor. Als Meßergebnisse entstehen Chromatogramme der in den Figuren 1 - 6 und 8 dargestellten Form. In den Chromatogrammen ist stets die Detektorsignalintensität über der Zeit aufgetragen.

Die Identifizierung eines Verschnittpolymers erfolgt erfindungsgemäß anhand zumindest eines charakteristischen Peaks eines Hauptbruchstücks des Polymers. Fig. 1 zeigt zunächst das Chromatogramm der Pyrolyseprodukte eines reinen Bitumens; die folgenden Fign. zeigen die erfindungsgemäß erhaltenen Chromatogramme der Pyrolyseprodukte von Bitumen/Polymer-Mischungen, aus denen die geeigneten Hauptbruchstücke der Polymeren für die Identifizierung zu entnehmen sind. Fig. 2 zeigt das Chromatogramm einer Bitumen-Mischung mit 7 Gew.-% eines Terpolymeren aus 7 Gew.-% Ethen, 67 Gew.-% Propen und 26 Gew.-% Buten-1, Fig. 3 das Chromatogramm von Bitumen mit 20 Gew.-% eines ataktischen Polypropens, Fig. 4 das Chromatogramm von Bitumen mit 5 Gew.-% eines Styrol-Butadien-Styrol-Blockcopolymers und Fig. 5 das Chromatogramm von Bitumen mit 15 Gew.-% eines Terpolymeren aus 14% Ethen, 72% Propen und 14% Buten-1.

Der quantitative Polymergehalt der Mischung kann erfindungsgemäß vorzugsweise anhand der Peakflächen der charakteristischen Peaks mittels einer Kalibrierkurve bestimmt werden. Hierzu geeignete Kalibrierkurven geben vorzugsweise das Verhältnis der Peakfläche eines charakteristischen Polymerbruchstücks zur Peakfläche eines charakteristischen Bitumenbruchstücks in Abhängigkeit von der Polymerkonzentration in der Mischung wieder. Aus dem gemessenen Chromatogramm einer zu untersuchenden Probe unbekannter Zusammensetzung kann so zunächst das Verhältnis der charakteristischen Peakflächen als Kenngröße bestimmt und anhand der Kalibrierkurve die zugehörige Polymerkonzentration abgelesen werden. Bei der Erstellung geeigneter Kalibrierkurven sind auch andere Auftragungen gegebenenfalls unter Hinzuziehen weiterer Peakflächen und Peakflächenverhältnisse möglich, die sich für den Fachmann in naheliegender Weise aus der bevorzugten Form der Kalibrierkurve ableiten lassen. In diesen Fällen sind analog die zur quantitativen Bestimmung erforderlichen Kenngrößen aus den Chromatogrammen abzulesen.

Die Kalibrierkurven werden durch Untersuchungen von Bitumen/Polymer-Mischungen bekannten Polymergehalts bestimmt. Die Bitumen/Polymer-Mischungen bekannten Polymergehalts werden im allgemeinen hergestellt durch Aufschmelzen des Bitumens und Einrühren des Polymergranulates. Die Mischung wird anschließend auf eine Folie gegossen und erkalten gelassen. Die Probe wird schließlich analog zum erfindungsgemäßen Verfahren einer Pyrolyse-Gaschromatographie unterzogen, aus der die gewünschte Kenngröße ermittelt wird, die zur Aufstellung der Kalibrierkurve benötigt wird.

Das erfindungsgemäße Verfahren wird durch die nachfolgenden Beispiele näher erläutert, ohne darauf beschränkt zu sein:

### Beispiel 1: Identifizierung von Polyolefinen mit unterschiedlicher Monomerzusammensetzung

Polyolefine mit unterschiedlicher Monomerzusammensetzung können aufgrund des unterschiedlichen Monomerverhältnisses identifiziert werden.

Es wurden zwei ataktische Terpolymere aus Ethen, Propen und Buten eingesetzt mit jeweils einem 15 Gew.-%-Anteil in der Mischung mit Bitumen B 200 mit einer Penetration im Bereich von 160 bis 210 (1/10 mm) und einem Erweichungspunkt im Bereich von 37 bis 44 °C.
- Polymer A:: 14 Gew.-% Ethen, 72 Gew.-% Propen und 14 Gew.-% Buten-1 Verhältnis Propen/Buten-1 = 5,14,
- Polymer B:: 7 Gew.-% Ethen, 67 Gew.-% Propen und 26 Gew.-% Buten-1 Verhältnis Propen/Buten-1 = 2,58.

Die Mischungsherstellung und Pyrolyse-Gaschromatographie erfolgte wie unter Beispiel 2 beschrieben.

Zur Auswertung wurde das Verhältnis von zwei für die Terpolymeren charakteristischen Peaks bzw. Peakmultipletts gebildet. Diese Peaks können nach einem Vergleich mit den Chromatogrammen der reinen Polymeren deren Fragmenten zugeordnet werden; es sind ein Einzelpeak mit der Retentionszeit von 4,3 Minuten und ein Dublett mit der Retentionszeit von 5,3 Minuten.

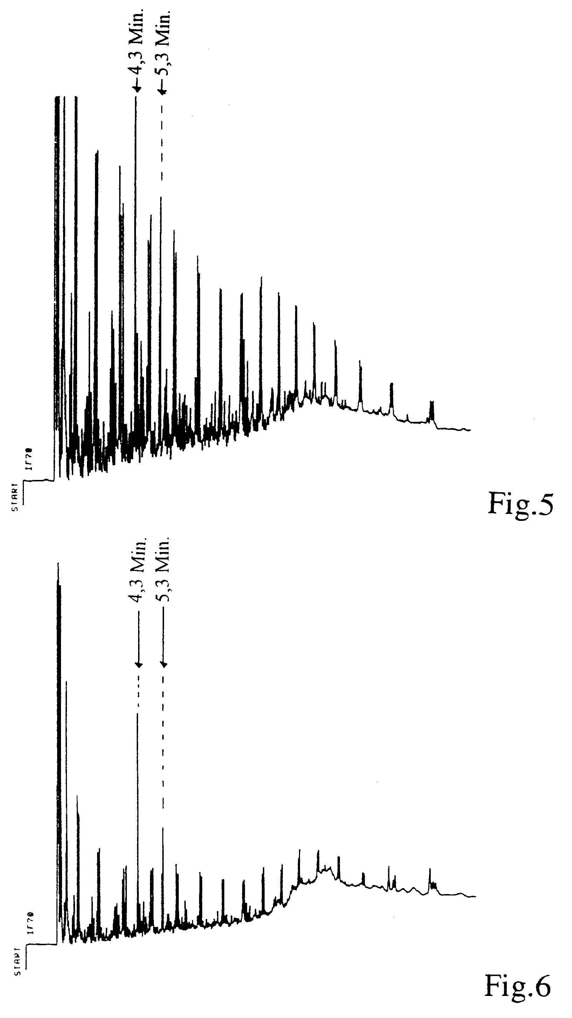

Für Polymer A beträgt das Verhältnis der Peakflächen 2,3 (s. Fig. 5), für Polymer B beträgt das Verhältnis 1,2 (s. Fig. 6).

Anhand dieser unterschiedlichen charakteristischen Peakverhältnisse ist es also möglich, die Terpolymere A und B in einer Bitumen/Polymer-Mischung zu identifizieren.

### Beispiel 2: Erstellung einer Kalibrierreihe

Bitumen B 80 mit einer Penetration im Bereich von 70 bis 100 (1/10 mm) und einem Erweichungspunkt im Bereich von 44 bis 49 °C wurde auf 180 - 200 °C erwärmt, und verschiedenen Proben wurden jeweils 3, 5, 7, 9, 15 und 20 Gew.-% eines Terpolymeren aus 7 Gew.-% Ethen, 67 Gew.-% Propen und 26 Gew.-% Buten-1 in Granulatform unter Rühren zugegeben. Die Mischungen wurden auf Cellophanfolie zum Erkalten ausgegossen.

Von diesen Mischungen wurden jeweils 0,5 - 1 mg auf einen Draht mit der Curie-Temperatur von 610 °C geklemmt und dieser in den Hochfrequenzofen eingeführt. Die Pyrolysedauer wurde auf 3 Sekunden eingestellt, die Temperatur der Transferleitung betrug 200 °C, die des Einspritzblocks 250 °C. Trägergas war Helium.

Die gaschromatische Trennung wurde auf der Säule HP 5/Hewlett Packard durchgeführt: 5 % Diphenyldimethylpolysiloxan, Länge 25 m, Innendurchmesser 0,32 mm und Filmdicke 0,52 µm mit dem Temperaturprogramm: 40 °C-1 Minute isotherm, dynamisch auf 230 °C mit 20 K/Min., 230 °C-10 Min. isotherm. Split 1 : 50. Eingesetzt wurde ein Hewlett Packard HP 5890 und ein Flammenionisationsdetektor.

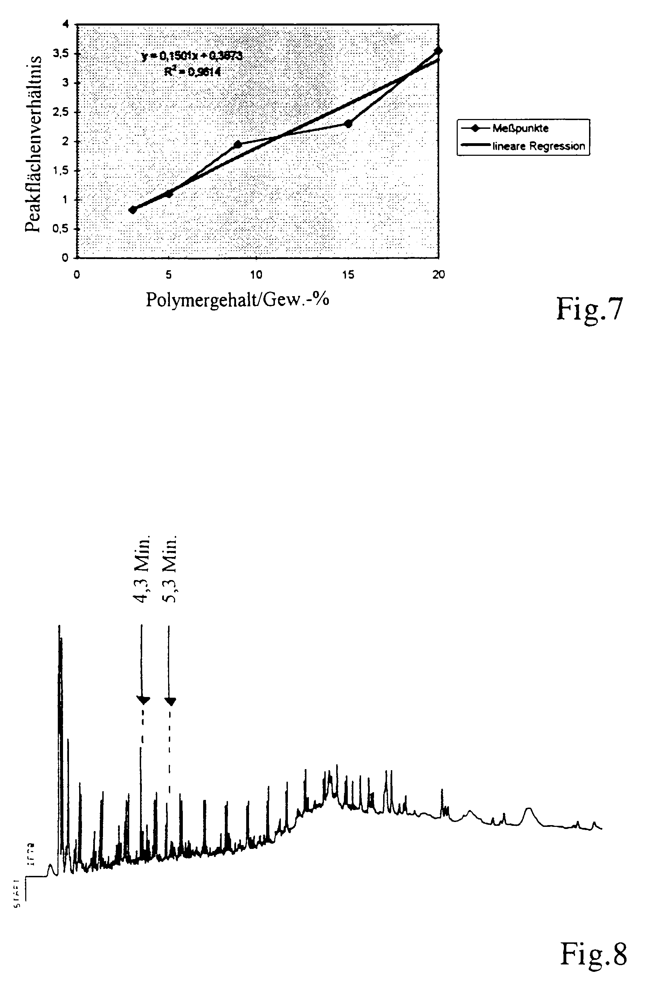

Zur Auswertung wurden charakteristische Peaks ausgewählt: für Bitumen der dritte Peak eines Tripletts mit der Retentionszeit 3,84 Minuten, für das Polymer der Peak bei 4,3 Minuten. Das Verhältnis von Peakfläche Polymerbruchstück zu Peakfläche Bitumenbruchstück ergibt aufgetragen gegen den eingesetzten Polymeranteil die gesuchte Kalibrierfunktion, die in Fig. 7 dargestellt ist.

### Beispiel 3: Untersuchung einer Bitumen/Polymer-Mischung

Die Probe einer Bitumen/Polymer-Mischung unbekannter Zusammensetzung wurde erfindungsgemäß qualitativ und quantitativ untersucht (siehe Figur 8).

Die unbekannte Probe zeigt zusätzlich zu dem Bitumenfingerprint 2 Peaks, einen Peak mit der Retentionszeit 4,3 Minuten und ein Dublett mit der Retentionszeit 5,3 Minuten. Diese Fragmente sind charakteristisch für amorphe Polyolefine. Das Peakflächenverhältnis entspricht mit 1,1 dem des Polymertyps B (vgl. Figur 6). Es handelt sich also beim Modifier um das Polymer B aus Beispiel 1.

Zur quantitativen Auswertung wurden folgende Peaks ausgewählt: für Bitumen der dritte Peak eines Tripletts mit der Retentionszeit 3,84 Minuten und für das Polymer der Peak bei 4,3 Minuten. Das Peakflächenverhältnis beträgt 1,44. Wird die Kalibrierkurve dieses Polymeren B zugrundegelegt (vgl. Figur 7), ergibt sich ein Gehalt von etwa 7 Gew.-% im Bitumen, berechnet als Polymer B mit einem Propen/Buten-1-Verhältnis von 2,6.

## Patentansprüche

1. Verfahren zur qualitativen und/oder quantitativen Bestimmung des Polymergehalts in Bitumen/Polymer-Mischungen, wobei das zu untersuchende Polymer kein Homopolyethylen ist,
dadurch gekennzeichnet,
daß eine Probe der Mischung in inerter Atmosphäre pyrolysiert wird und die Pyrolyseprodukte on-line gaschromatographisch analysiert werden.

2. Verfahren nach Anspruch 1,
dadurch gekennzeichnet,
daß die qualitative Bestimmung des Polymers durch das Erkennen von Peaks charakteristischer Polymer-Pyrolysebruchstücke im Chromatogramm erfolgt, die sich deutlich vom Bitumenfragmentierungsmuster unterscheiden.

3. Verfahren nach zumindest einem der vorherigen Ansprüche,
dadurch gekennzeichnet,
daß quantitative Bestimmungen anhand von aus dem Chromatogramm abgelesenen Kenngrößen mit Hilfe zumindest einer geeigneten Kalibrierfunktion, die den Zusammenhang zwischen den Kenngrößen und der Zusammensetzung der Mischung wiedergibt, durchgeführt werden.

4. Verfahren nach Anspruch 3,
dadurch gekennzeichnet,
daß als Kenngröße das Verhältnis der Peakflächen zumindest eines charakteristischen Polymerbruchstückpeaks und zumindest eines charakteristischen Bitumenbruchstückpeaks gebildet wird.

5. Verfahren nach zumindest einem der vorherigen Ansprüche,
dadurch gekennzeichnet,
daß die Pyrolysedauer weniger als 10 s beträgt.

6. Verfahren nach Anspruch 5,
dadurch gekennzeichnet,
daß die Pyrolysedauer 0,01 bis 5 s beträgt.

7. Verfahren nach zumindest einem der vorherigen Ansprüche,
dadurch gekennzeichnet,
daß die Pyrolyse im Temperaturbereich von 400 °C bis 900 °C durchgeführt wird.

8. Verfahren nach Anspruch 7,
dadurch gekennzeichnet,
daß die Pyrolyse im Temperaturbereich von 500 °C bis 800 °C durchgeführt wird.

9. Verfahren nach zumindest einem der vorherigen Ansprüche,
dadurch gekennzeichnet,
daß ein Trägergasstrom über die zu pyrolysierende Probe geleitet wird, der die Pyrolyseprodukte direkt in den Gaschromatographen überführt.

10. Verfahren nach zumindest einem der vorherigen Ansprüche,
dadurch gekennzeichnet,
daß bei der on-line Gaschromatographie Kapillarsäulen verwendet werden.

11. Verfahren nach Anspruch 10,
dadurch gekennzeichnet,
daß eine Glaskapillare mit einer stationären Phase aus Diphenyldimethylpolysiloxan verwendet wird.
